(19) Europäisches Patentamt
European Patent Office
Office européen des brevets

(11) Veröffentlichungsnummer: **0 031 915**
**B1**

(12) **EUROPÄISCHE PATENTSCHRIFT**

(45) Veröffentlichungstag der Patentschrift: **25.05.83**

(51) Int. Cl.³: **C 07 C 69/74,** C 07 C 121/48, C 07 C 49/567, C 07 C 21/19 // C07F9/40

(21) Anmeldenummer: **80107716.5**

(22) Anmeldetag: **08.12.80**

(54) **Verfahren zur Herstellung von Halogenalkenen.**

(30) Priorität: **03.01.80 DE 3000065**

(43) Veröffentlichungstag der Anmeldung: **15.07.81 Patentblatt 81/28**

(45) Bekanntmachung des Hinweises auf die Patenterteilung: **25.05.83 Patentblatt 83/21**

(84) Benannte Vertragsstaaten: **BE CH DE FR GB IT LI NL**

(56) Entgegenhaltungen:
**DE-A-2 827 101**
**DE-A-2 922 961**

(73) Patentinhaber: **BAYER AG, Zentralbereich Patente, Marken und Lizenzen, D-5090 Leverkusen 1, Bayerwerk (DE)**

(72) Erfinder: **Hoffmann, Hellmut, Prof. Dr., Tersteegenweg 17, D 5600 Wuppertal-1 (DE)**

**EP 0 031 915 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von Halogenalkenen

Die Erfindung betrifft ein neues Verfahren zur Herstellung von teilweise bekannten Halogenalkenen.

Es ist bekannt, daß man bestimmte Halogenalkene, wie z. B. 3-(2-Chlor-2-phenyl-vinyl)-2,2-dimethyl-cyclopropan-1-carbonsäure-ethylester, erhält, wenn man Phosphonsäureester, wie z. B. Benzylphosphonsäurediethylester, mit starken Basen, wie z. B. Butyllithium, behandelt und die intermediär gebildeten Ylide mit Halogenüberträgern, wie z. B. Tetrachlorkohlenstoff, und dann mit Oxoverbindungen, wie z. B. 3-Formyl-2,2-dimethyl-cyclopropan-1-carbonsäure-ethylester, umsetzt (vergleiche US-PS 4 157 447).

Die Durchführung dieses Herstellungsverfahrens für Halogenalkene würde jedoch einen hohen technischen Aufwand erfordern.

Die zur Ylidbildung aus Phosphonsäureestern benötigten starken Basen, wie z. B. Butyllithium, sind feuchtigkeits- und luftempfindlich; daher wird die Reaktion in einer Inertgasatmosphäre, beispielsweise unter Stickstoff oder Argon und unter Verwendung eines inerten, sorgfältig getrockneten Verdünnungsmittels durchgeführt. Da die Umsetzung bei tiefer Temperatur, etwa bei −70° C zu erfolgen hat, muß das Reaktionsgemisch auch intensiv gekühlt werden. Die Aufarbeitung zur Isolierung der gewünschten Produkte, welche man als Isomerengemische erhält, ist ebenfalls aufwendig; sie umfaßt mehrere Extraktionen und Lösungsmitteldestillationen sowie einen chromatographischen Trennungsprozeß. Aus diesen Gründen ist die bekannte Synthesemethode für eine Herstellung von Halogenalkenen, wie 3-(2-Chlor-2-phenyl-vinyl)-2,2-dimethyl-cyclopropan-1-carbonsäureestern im industriellen Maßstab wenig geeignet.

Aus DE-OS 2 922 961 ist bekannt, daß die Umsetzung eines Triphenylphosphoniumsalzes mit einem 1-Formyl-but-2-encarbonsäureester in Anwesenheit einer starken Base zum Formylcyclopropancarbonsäureester führt.

Gemäß einer am Anmeldetag vorliegender Anmeldung noch nicht zum veröffentlichten Stand der Technik gehörenden Anmeldung der Anmelderin werden Alkene durch Umsetzung von α-Hydroxyphosphonsäureestern mit phosphorhaltigen Olefinierungsmitteln erhalten (DE-OS 2 917 620).

Es wurde nun ein Verfahren zur Herstellung von Halogenalkenen der Formel I

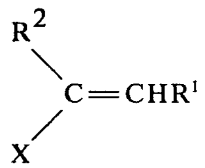

(I)

gefunden, in welcher

$R^1$ für gegebenenfalls halogen-substituiertes $C_1-C_4$-Alkyl, für $C_2-C_6$-Alkenyl, für gegebenenfalls durch Halogen, Cyano, Carbamoyl, $C_1-C_4$-Alkyl, $C_1-C_4$-Alkyl-carbonyl, $C_1-C_4$-Alkoxy-carbonyl und/oder Phenoxybenzyloxy-carbonyl (welches gegebenenfalls durch Fluor, Cyano und/oder Ethinyl substituiert ist) substituiertes $C_3-C_6$-Cycloalkyl, für gegebenenfalls durch Chlor substituiertes Benzyl oder Phenylethyl, oder für gegebenenfalls durch Halogen, $C_1-C_4$-Alkyl und/oder $C_1-C_4$-Alkoxy substituiertes Phenyl steht,

$R^2$ für Chlor, Brom, Alkyl, Alkenyl oder Alkinyl, jeweils mit bis zu 5 C-Atomen oder für Phenyl, welches gegebenenfalls durch Halogen, $C_1-C_4$-Alkyl, $C_1-C_2$-Halogenalkyl,$C_1-C_4$-Alkoxy,$C_1-C_2$-Halogenalkoxy, $C_1-C_2$-(Halo)Alkylendioxy, $C_1-C_4$-Alkylthio und/oder $C_1-C_2$-Halogenalkylthio substituiert ist, steht, und

X für Halogen steht,

bei welchem man α-Hydroxy-phosphonsäureester der Formel II

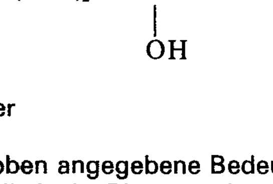

(II)

in welcher

$R^1$ die oben angegebene Bedeutung hat und

$R^3$ für Alkyl oder Phenyl steht oder die beiden Reste $R^3$ zusammen für geradkettiges oder verzweigtes Alkandiyl (Alkylen) stehen,

mit phosphorhaltigen Olefinierungsmitteln der Formel III

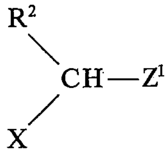

(III)

in welcher

$R^2$ und X die oben angegebenen Bedeutungen haben und
$Z^1$ für einen phosphorhaltigen Rest

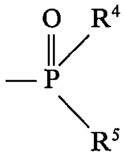 oder $-P(R^6)_3^{\oplus}$ $X^{\ominus}$

steht, worin

$R^4$ und $R^5$ gleich oder verschieden sind und einzeln für Alkyl, Phenyl, Alkoxy oder Phenoxy oder zusammen für Alkandioxy stehen,
$R^6$ für Alkyl oder Phenyl steht und
X für Halogen steht,

in Gegenwart von Basen, gegebenenfalls in Gegenwart von Katalysatoren, und gegebenenfalls unter Verwendung von Verdünnungsmitteln bei Temperaturen zwischen −70 und +150°C umsetzt, dadurch gekennzeichnet, daß man in Gegenwart von Halogenüberträgern arbeitet.

Überraschenderweise können nach dem neuen Verfahren, welches einfach und kostengünstig durchzuführen ist und für welches ohne großen Aufwand herzustellende Ausgangsverbindungen einzusetzen sind, Alkene der Formel (I) in guten Ausbeuten und in hoher Reinheit erhalten werden.

Vorteile des neuen Verfahrens liegen weiter beispielsweise in der Möglichkeit, die Umsetzung bei Raumtemperatur oder zumindest bei nicht weit davon abweichenden Temperaturen durchzuführen, billige Basen wie Alkali-hydroxide oder -alkoholate zu verwenden und wasserhaltige Lösungsmittel einzusetzen.

Ein besonderer Vorteil des erfindungsgemäßen Verfahrens besteht darin, daß die Herstellung von Aldehyden, welche im allgemeinen als Reaktionskomponente bei der Herstellung von Alkenen durch Umsetzung von Carbonylverbindungen mit phosphorhaltigen Olefinierungsmitteln eingesetzt werden, vermieden werden kann.

Verwendet man als Ausgangsstoffe beispielsweise α-(3-Methoxy-carbonyl-2,2-dimethyl-cycloprop-1-yl)-α-hydroxymethan-phosphonsäure-dimethylester und α-Chlorbenzylphosphonsäure-diethylester, so kann die erfindungsgemäße Reaktion dieser Verbindungen durch folgendes Formelschema skizziert werden:

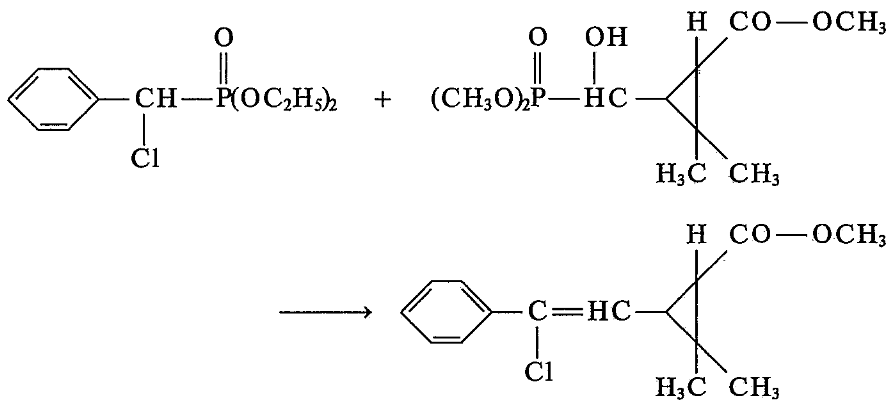

Die als Ausgangsstoffe zu verwendenden α-Hydroxy-phosphonsäureester sind durch Formel (II) definiert. Vorzugsweise steht darin

$R^3$ für $C_1-C_4$-Alkyl oder für Phenyl, oder beide Reste $R^3$ stehen gemeinsam für 2,2-Dimethyl-propan-1,3-diyl.

Bei einer besonders bevorzugten Gruppe von Ausgangsverbindungen der Formel (II) steht in dieser Formel

$R^1$ für den Rest

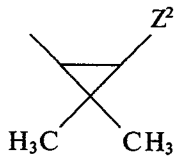

worin $Z^2$ für Cyano, Carbamoyl, Acetyl oder $C_1-C_4$-Alkoxycarbonyl steht und

$R^3$ für Methyl oder Ethyl steht.

Als Beispiele seien genannt:

α-Hydroxy-3-methyl-benzyl-, α-Hydroxy-4-methyl-benzyl-, α-Hydroxy-3,4-dimethyl-benzyl-, α-Hydroxy-4-fluor-benzyl-, α-Hydroxy-3-chlor-benzyl-, α-Hydroxy-4-chlor-benzyl-, α-Hydroxy-3,4-dichlor-benzyl-, α-Hydroxy-3-brom-benzyl-, α-Hydroxy-4-brom-benzyl-, α-Hydroxy-4-methoxy-benzyl- und α-Hydroxy-3,4-dimethoxy-benzyl-phosphonsäure-dimethylester und -diethylester;
α-Hydroxy-α-(3-methoxycarbonyl-2,2-dimethyl-cycloprop-1-yl)-methanphosphonsäure-dimethylester und -diethylester,
α-Hydroxy-α-(3-ethoxycarbonyl-2,2-dimethyl-cycloprop-1-yl)-methanphosphonsäure-dimethylester und -diethylester,
α-Hydroxy-α-(3-acetyl-2,2-dimethyl-cycloprop-1-yl)-methanphosphonsäuredimethylester und -diethylester und
α-Hydroxy-α-(3-cyano-2,2-dimethyl-cycloprop-1-yl)-methanphosphonsäuredimethylester und -diethylester.

Die Ausgangsverbindungen der Formel (II) sind zum Teil noch nicht in der Literatur beschrieben, können aber nach im Prinzip bekannten Verfahren hergestellt werden.

So erhält man substituierte α-Hydroxy-methanphosphonsäureester der Formel (II), indem man die entsprechenden Oxoverbindungen der Formel IV

$$R^1-CO-\overset{O}{\overset{\|}{P}}(OR^3)_2 \qquad (IV)$$

in welcher

$R^3$ und $R^1$ die oben angegebenen Bedeutungen haben,

mit einem Reduktionsmittel wie z. B. Natriumtetrahydridoborat (Natriumboranat), gegebenenfalls unter Verwendung eines Verdünnungsmittels wie z. B. Wasser oder wäßriges Methanol, bei Temperaturen zwischen $-20$ und $+50°$ C, vorzugsweise zwischen $-10$ und $+30°$ C, umsetzt und durch Zugabe eines Puffermittels wie z. B. Natriumhydrogenphosphat den pH-Wert zwischen 5 und 8 hält (vergleiche Chem. Ber. 103 (1970), 2984—2986).

Zur Aufarbeitung und Isolierung der Produkte extrahiert man mit einem mit Wasser nicht mischbaren Lösungsmittel wie z. B. Methylenchlorid, trocknet die organische Phase, filtriert und destilliert das Lösungsmittel unter vermindertem Druck ab.

Die substituierten α-Oxo-methanphosphonsäureester der Formel (IV) sind zum Teil nicht in der Literatur beschrieben. Man erhält diese Verbindungen durch Umsetzung von Carbonsäurechloriden der Formel V

$$R^1-CO-Cl \qquad (V)$$

in welcher

$R^1$ die oben angegebene Bedeutung hat,

mit Phosphorigsäureestern der Formel VI

$$R^7O-P(OR^3)_2 \qquad (VI)$$

in welcher

$R^3$ die oben angegebene Bedeutung hat und
$R^7$ für Alkyl, insbesondere für Methyl oder Ethyl steht,

bei Temperaturen zwischen −20 und +150° C, vorzugsweise zwischen 0 und 120° C (vergleiche J. Am. Chem. Soc. 86 (1964), 3862—3866 und Methoden der organischen Chemie (Houben-Weyl-Müller), 4. Aufl. Band 12/1, S. 453, Georg-Thieme-Verlag, Stuttgart 1963).

Verbindungen der Formeln (V) und (VI) sind bekannt. Die noch nicht bekannten Cyclopropancarbonsäure-chloride der Formel (Va) erhält man gemäß nachstehendem Reaktionsschema aus bekannten Cyclopropan-carbonsäureestern (vergleiche J. Am. Chem. Soc. 89 (1967), 3912—3914; J. Org. Chem. 32 (1967), 3351—3355; Tetrahedron Lett. 1978, 1847—1850; Bull. Soc. Chim. Belg. 87 (1978), 721—732) nach an sich bekannten Methoden, indem man zunächst durch Hydrolyse, beispielsweise durch Umsetzung mit wäßrig-alkoholischer Kalilauge bei Temperaturen zwischen 20 und 100°C und anschließendes Ansäuern die entsprechenden Cyclopropancarbonsäuren herstellt und diese mit Halogenierungsmitteln, wie z. B. Thionylchlorid, bei Temperaturen zwischen 20 und 80° C umsetzt:

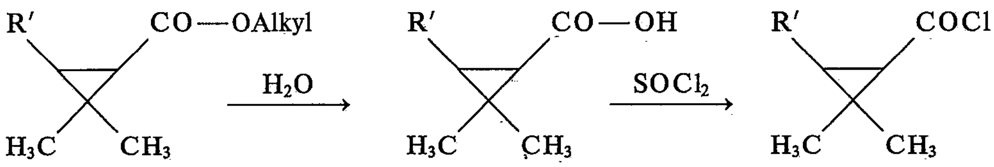


(Va)

Die weiter als Ausgangsstoffe zu verwendenden phosphorhaltigen Olefinierungsmittel sind durch Formel (III) definiert. Vorzugsweise stehen darin

X für Chlor oder Brom, und
$Z^1$ für den Rest

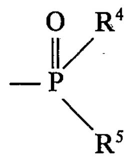

worin $R^4$ und $R^5$ gleich oder verschieden sind und einzeln für $C_1$—$C_4$-Alkoxy, Phenoxy oder zusammen für 2,2-Dimethyl-propan-1,3-dioxy stehen.

Als Beispiele seien genannt:

Dichlormethanphosphonsäuredimethylester und -diethylester, α-Chlor-benzylphosphonsäure-dimethylester und -diethylester, α-Brom-benzylphosphonsäure-dimethylester und -diethylester, 4-Fluor-, 4-Chlor-, 3-Chlor-, 3,4-Dichlor-, 3-Brom-, 4-Brom-, 3-Methyl-, 4-Methyl-, 3,4-Dimethyl-, 4-Methoxy- und 3,4-Dimethoxy- -α-chlor-benzylphosphonsäuredimethylester und -diethylester; 4-Fluor-, 3-Chlor-, 4-Chlor-, 3,4-Dichlor-, 3-Brom-, 4-Brom-, 3-Methyl-, 4-Methyl-, 3,4-Dimethyl-, 4-Methoxy- und 3,4-Dimethoxy- -α-brom-benzylphosphonsäure-dimethylester und -diethylester.

Die phosphorhaltigen Olefinierungsmittel der Formel (III) sind zum Teil noch nicht in der Literatur beschrieben, können aber nach im Prinzip bekannten Verfahren hergestellt werden.

Unter Formel (III) fallende α-Chlor- und α-Bromphosphonsäureester erhält man durch Umsetzung von α-Hydroxy-phosphonsäureestern der Formel VII

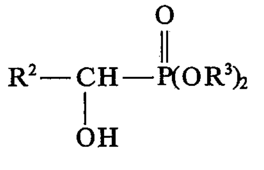

(VII)

in welcher

$R^2$ und $R^3$ die oben angegebenen Bedeutungen haben,

mit einem Chlorierungsmittel, wie z. B. Thionylchlorid, bzw. mit einem Bromierungsmittel, wie z. B. Dibromtriphenylphosphoran, gegebenenfalls in Gegenwart eines Säureakzeptors, wie z. B. Pyridin, und gegebenenfalls unter Verwendung eines Verdünnungsmittels, wie z. B. Methylenchlorid oder Ethylenchlorid, bei Temperaturen zwischen 10 und 100° C bzw. zwischen −50 und +50° C (vergleiche J.

Am. Chem. Soc. 87 (1965), 2777—2778; Chimia 28 (1974), 656—657).

α-Hydroxy-phosphonsäureester der Formel (VII) sind bereits bekannt (vergleiche DE-OS 2 827 101).

Das erfindungsgemäße Verfahren zur Herstellung von Alkenen der Formel (I) wird vorzugsweise unter Verwendung geeigneter Lösungs- oder Verdünnungsmittel durchgeführt. Als solche kommen neben Wasser praktisch alle inerten organischen Solventien in Frage. Hierzu gehören insbesondere aliphatische und aromatische, gegebenenfalls chlorierte Kohlenwasserstoffe, wie Pentan, Hexan, Heptan, Cyclohexan, Benzol, Toluol, Xylol, Methylenchlorid, Ethylenchlorid, Chloroform, Tetrachlorkohlenstoff, Chlorbenzol und Dichlorbenzole, Ether, wie Diethyl- und Dibutylether, Tetrahydrofuran und Dioxan, Alkohole wie Methanol, Ethanol, n- und iso-Propanol, n-, iso-, sek.- und tert.-Butanol sowie aprotisch polare Lösungsmittel wie Dimethylformamid und Dimethylsulfoxid.

Bei Arbeiten im Zweiphasenmedium werden neben etwa 50%iger Natronlauge oder Kalilauge organische Lösungsmittel wie Pentan, Hexan, Heptan, Benzol oder Toluol verwendet, welche mit Wasser nicht mischbar sind.

Als Katalysatoren werden — bei Durchführung des erfindungsgemäßen Verfahrens in mehrphasigen Reaktionsmedien — Verbindungen verwendet, welche gewöhnlich bei Reaktionen in mehrphasigen Systemen als Hilfsmittel zum Phasentransfer von Reaktanden dienen. Insbesondere seien als solche Phasentransfer-Katalysatoren Tetraalkyl- und Trialkyl-benzyl-ammoniumsalze, wie z. B. Tetrabutylammoniumbromid und Trimethylbenzyl-ammoniumchlorid, genannt.

Als Basen können bei der Durchführung des erfindungsgemäßen Verfahrens die bei Carbonyl-Olefinierungen üblichen Basen verwendet werden. Genannt seien Alkalihydroxide wie z. B. Kalium- und Natrium-hydroxid, Alkalialkoholate wie z. B. Kalium- und Natriummethylat, -ethylat, -n- und -iso-propylat, -n-, -iso-, -sek.- und -tert.-butylat, Alkalihydride wie z. B. Natriumhydrid sowie Alkyllithiumverbindungen wie z. B. Butyllithium. Vorzugsweise werden die genannten Alkalihydroxide und/oder -alkoholate verwendet.

Das erfindungsgemäße Verfahren wird unter Verwendung von Halogenüberträgern durchgeführt. Als solche kommen insbesondere Verbindungen der Formel VIII

$$R^8—CX_3 \qquad \text{(VIII)}$$

in welcher

$R^8$ für Halogen, insbesondere Chlor oder Brom, gegebenenfalls halogen-substituiertes Alkyl, insbesondere Chlormethyl, oder gegebenenfalls halogen-substituiertes Phenyl steht und

X für Halogen, insbesondere Chlor oder Brom steht,

in Betracht.

Als Beispiele für die Halogenüberträger der Formel (VIII) seien genannt:

Tetrachlorkohlenstoff, Tetrabromkohlenstoff, Hexachlorethan, Benzotrichlorid und 4-Chlor-benzotrichlorid. Tetrachlorkohlenstoff wird besonders bevorzugt.

Das Verfahren wird im allgemeinen bei Normaldruck durchgeführt. Die Reaktionstemperaturen liegen zwischen −70 und +150° C, vorzugsweise zwischen −20 und +50° C.

Zur Durchführung des erfindungsgemäßen Verfahrens werden die Ausgangsverbindungen der Formeln (II) und (III) und der Halogenüberträger der Formel (VIII) gewöhnlich in angenähert äquimolaren Mengen eingesetzt. Bei Arbeiten in einphasigen Systemen verwendet man im allgemeinen zwei Basenäquivalente, bei Verwendung von 50%igen Alkalilaugen als Zweitphasen im allgemeinen das 5- bis 15fache der stöchiometrisch erforderlichen Menge.

Man legt die Base und gegebenenfalls den Katalysator in einem geeigneten Reaktionsmedium vor und gibt dazu gleichzeitig oder nacheinander — gegebenenfalls in einem der angegebenen Solventien gelöst — die Ausgangstoffe der Formeln (II) und (III) sowie den Halogenüberträger. Zur Vervollständigung der Reaktion wird das Gemisch mehrere Stunden gerührt.

Die Aufarbeitung kann nach üblichen Methoden erfolgen. Zur Isolierung destillierbarer Produkte kann man beispielsweise das Lösungsmittel abdestillieren, den Rückstand in einem mit Wasser nicht mischbaren Lösungsmittel, wie z. B. Ligroin oder Toluol, aufnehmen, die Lösung mit Wasser waschen, trocknen, filtrieren und destillieren. Zur Isolierung kristalliner Produkte kann man gegebenenfalls das Reaktionsgemisch in (Eis)-Wasser gießen und vom kristallinen Produkt absaugen, oder man kann das Reaktionsgemisch — falls das Lösungsmittel nicht mit Wasser mischbar ist — mit Wasser waschen, trocknen, filtrieren und eindampfen. Nach der letztgenannten Aufarbeitungsmethode können auch ölige, schwer destillierbare Produkte relativ rein erhalten werden.

Die Charakterisierung der Produkte erfolgt durch ihre Schmelz- oder Siedepunkte.

Nach dem erfindungsgemäßen Verfahren herzustellende Verbindungen der Formel (I) können als Zwischenprodukte zur Herstellung von Insektiziden verwendet werden (vergleiche DE-OS 2 738 150).

Beispiel

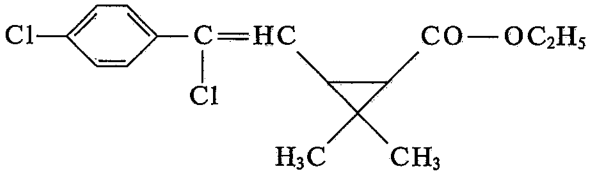

60 g (0,2 Mol) α-Chlor-4-chlor-benzyl-phosphonsäure-diethylester werden zu einer auf −10° C gekühlten Lösung von 21 g (0,4 Mol) Natriumethylat in 200 ml Ethanol und 200 ml Tetrahydrofuran im Laufe von 10 Minuten gegeben und das Gemisch wird noch 20 Minuten bei −10° C gerührt. Dann wird eine Lösung von 58 g (0,2 Mol) α-Hydroxy-α-(3-ethoxycarbonyl-2,2-dimethyl-1-cyclopropyl)-methan-phosphonsäure-dimethylester in 31 g Tetrachlorkohlenstoff bei einer Innentemperatur von −10 bis 0° C im Laufe von 20 Minuten dazu gegeben. Das Reaktionsgemisch wird ca. 15 Stunden bei 20° C gerührt, in Wasser gegossen und mit Toluol extrahiert. Die organische Phase wird mit Wasser gewaschen, über Natriumsulfat getrocknet, filtriert und destilliert. Man erhält 48 g (77% der Theorie) 3-(2-Chlor-2-(4-chlor-phenyl)-vinyl)-2,2-dimethyl-cyclopropan-1-carbonsäureethylester vom Siedepunkt 115° C/0,01 mbar.

Analog erhält man auch 3-(2,2-Dichlor-vinyl)-2,2-dimethylcyclopropan-1-carbonsäure-ethylester, die entsprechenden Methylester, n- und iso-Propylester sowie n-, iso-, sek.- und tert.-Butylester und die nachstehend aufgeführten Verbindungen:

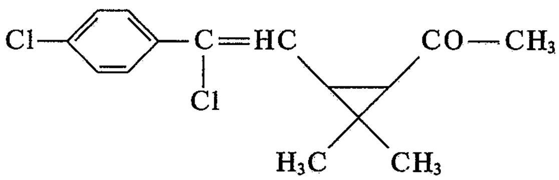

(3-(2-Chlor-2-(4-chlor-phenyl)-vinyl)-2,2-dimethyl-1-cyclopropyl)-methyl-keton;
Siedepunkt 90°C/0,01 mbar.

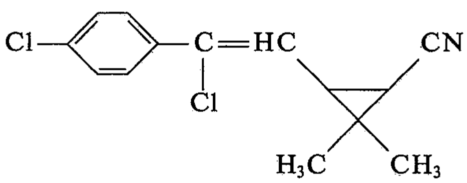

3-(2-Chlor-2-(4-chlor-phenyl)-vinyl)-2,2-dimethyl-cyclopropan-1-carbonsäurenitril;
Siedepunkt 94°C/0,01 mbar.

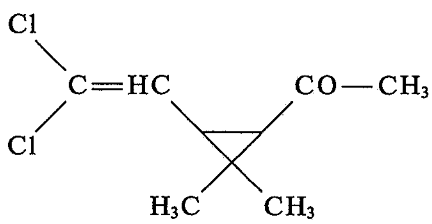

(3-(2,2-Dichlor-vinyl)-2,2-dimethyl-1-cyclopropyl)-methyl-keton;

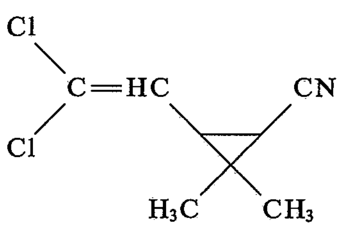

3-(2,2-Dichlor-vinyl)-2,2-dimethyl-cyclopropan-1-carbonsäurenitril;

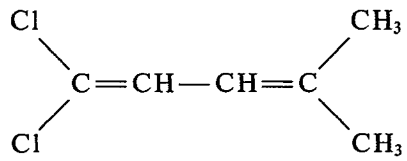

1,1-Dichlor-4-methyl-1,3-pentadien.

Die als Ausgangsstoffe zu verwendenden $\alpha$-Hydroxy-phosphonsäureester der Formel (II) können beispielsweise wie folgt hergestellt werden:

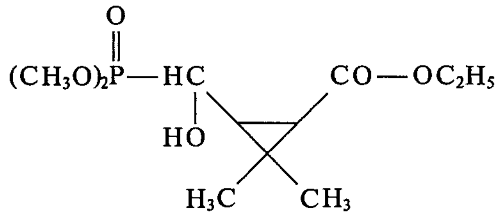

Eine Lösung von 56 g (0,2 Mol) $\alpha$-Oxo-$\alpha$-(3-ethoxycarbonyl-2,2-dimethyl-1-cyclopropyl)-methanphosphonsäure-dimethylester in 100 ml Methylenchlorid wird zu einer auf 0° C gekühlten Lösung von 2,5 g Natriumtetrahydridoborat in 100 ml Wasser tropfenweise gegeben und das Reaktionsgemisch wird noch 90 Minuten bei 0° C gerührt. Dann wird die wäßrige Phase von der organischen Phase abgetrennt und noch zweimal mit Methylenchlorid extrahiert. Die vereinigten organischen Phasen werden über Natriumsulfat getrocknet und filtriert. Vom Filtrat wird das Lösungsmittel unter vermindertem Druck sorgfältig abdestilliert. Man erhält 40 g (71% der Theorie) $\alpha$-Hydroxy-$\alpha$-(3-ethoxycarbonyl-2,2-dimethyl-1-cyclopropyl)-methanphosphonsäure-dimethylester als öligen Rückstand.

Analog erhält man:

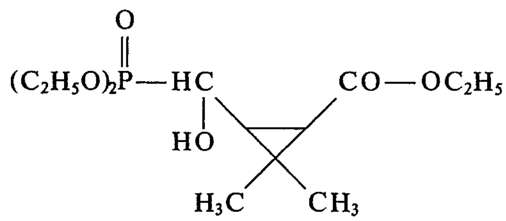

Schmelzpunkt: 62°C

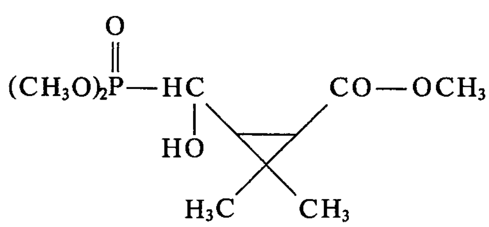

Schmelzpunkt: 69°C

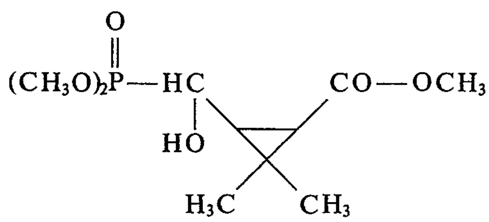

Schmelzpunkt: 104°C

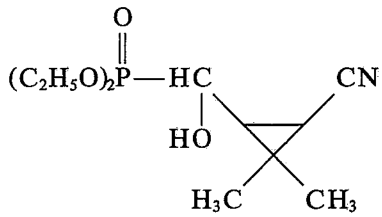

Als Ausgangsstoffe zu verwendende $\alpha$-Oxo-phosphonsäureester der Formel (IV) können beispielsweise wie folgt hergestellt werden:

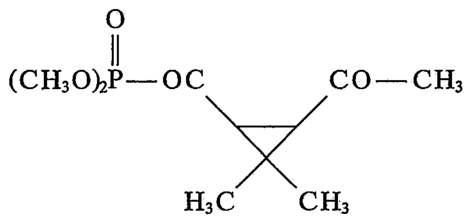

6,5 g (0,05 Mol) Trimethylphosphit werden zu einer auf 35 bis 40°C erwärmten Lösung von 9 g (0,05 Mol) 3-Acetyl-2,2-dimethyl-cyclopropan-1-carbonsäurechlorid in 20 ml Methylenchlorid tropfenweise gegeben und das Reaktionsgemisch wird 15 Stunden bei 15 bis 25°C gerührt. Nach Abdestillieren des Lösungsmittels im Vakuum erhält man 9 g (72% der Theorie) $\alpha$-Oxo-$\alpha$-(3-acetyl-2,2-dimethylcycloprop-1-yl)-methan-phosphonsäure-dimethylester.

Analog vorstehendem Beispiel erhält man

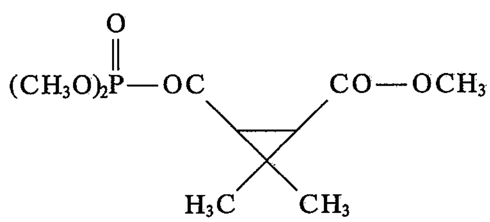

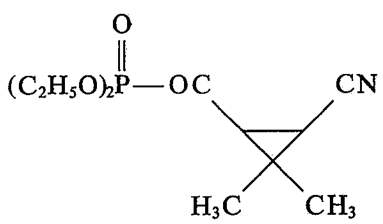

Als Ausgangsstoffe zu verwendende Cyclopropancarbonsäurechloride der Formel (Va) können beispielsweise wie folgt hergestellt werden:

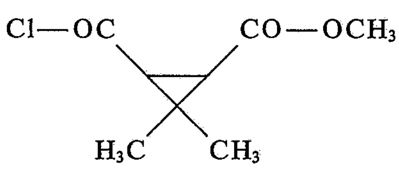

Eine Mischung aus 172 g (1 Mol) 3-Methoxycarbonyl-2,2-dimethyl-cyclopropan-1-carbonsäure, 130 g Thionylchlorid, 2 ml Dimethylformamid, und 200 ml Methylenchlorid wird 4 Stunden unter Rückfluß zum Sieden erhitzt. Man erhält nach Destillation im Vakuum 135 g (71% der Theorie) 3-Methoxycarbonyl-2,2-dimethyl-cyclopropan-1-carbonsäurechlorid vom Siedepunkt 86°C/15 mbar.

Analog erhält man

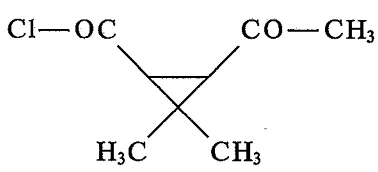

Als Ausgangsstoffe zu verwendende α-Halogen-benzylphosphonsäureester als phosphorhaltige Olefinierungsmittel der Formel (III) können beispielsweise wie folgt hergestellt werden:

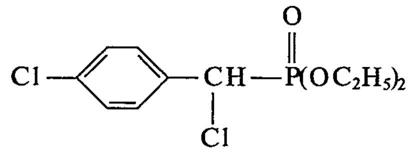

Einem Gemisch aus 20,2 g (0,0725 Mol) 4-Chlor-α-hydroxybenzylphosphonsäure-diethylester, 65 g Dichlormethan und 5,8 g (0,0725 Mol) Pyridin werden bei 20—40°C unter leichter Wasserkühlung innerhalb von etwa 1 Stunde 9,2 g (0,0768 Mol) Thionylchlorid hinzugesetzt. Das Reaktionsgemisch wird dann 3 Stunden unter Rückfluß erhitzt und 12 Stunden ohne weitere Wärmeeinwirkung nachgerührt. Das Gemisch wird auf etwa 100 g Eiswasser gegossen, die organische Phase abgetrennt und getrocknet. Nach Abdestillieren des Lösungsmittels wird der Rückstand bei 6 Torr und 45°C eingeengt. Man erhält 21 g (97,7% der Theorie) 4-Chlor-α-chlor-benzyl-phosphonsäurediethylester als gelbes viskoses Öl mit einer Reinheit von 98,6% (Gaschromatogramm) und einem Brechungsindex von $n_D^{24}$: 1,5250.

Analog erhält man

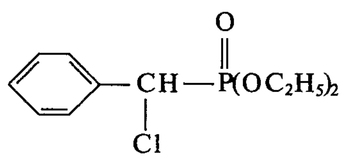

Brechungsindex: $n_D^{23}$: 1,5117

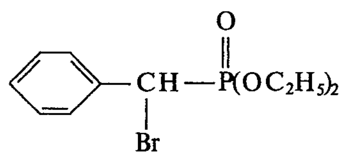

In eine Lösung von 131 g (0,5 Mol) Triphenylphosphin in 500 ml Methylenchlorid läßt man unter Feuchtigkeitsausschluß bei 30—35° C 90 g Brom gelöst in 250 ml Methylenchlorid zutropfen. Man rührt 1 Stunde bei Raumtemperatur nach, dann läßt man bei −20°C innerhalb 1 Stunde eine Lösung von 122 g (0,5 Mol) α-Hydroxybenzyl-phosphonsäurediethylester in 250 ml Methylenchlorid zutropfen, rührt 1 Stunde bei −20°C nach und tropft dann innerhalb von einer Stunde bei −20°C 40 g Pyridin, gelöst in 250 ml Methylenchlorid zu. Man läßt 20 Stunden nachrühren, wobei die Temperatur langsam bis +20°C ansteigt. Das Reaktionsgemisch wird in einen Birnenkolben überführt und das Lösungsmittel im Wasserstrahlvakuum abdestilliert. Der Rückstand wird mit einem Liter Ether ausgerührt und von dem nicht gelösten Triphenylphosphinoxyd abgesaugt. (Triphenylphosphinoxyd 120 g = 86% der Theorie). Die Mutterlauge im Vakuum einengen und den Rückstand im Hochvakuum destillieren. Man erhält 112 g (72% der Theorie) α-Brombenzylphosphonsäurediethylester als hellgelbes Öl mit einem Siedepunkt von 110° C/0,01 Torr und einer Reinheit von 95,9% (GC).

Analog erhält man:

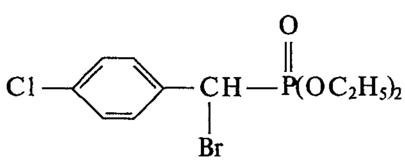

Siedepunkt: 125° C/0,01 Torr.

**Patentansprüche**

1. Verfahren zur Herstellung von Halogenalkenen der Formel I

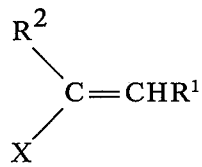

(I)

in welcher

$R^1$ für gegebenenfalls halogensubstituiertes $C_1-C_4$-Alkyl, für $C_2-C_4$-Alkenyl, für gegebenenfalls durch Halogen, Cyano, Carbamoyl, $C_1-C_4$-Alkyl, $C_1-C_4$-Alkyl-carbonyl, $C_1-C_4$-Alkoxy-carbonyl und/oder Phenoxybenzyloxy-carbonyl (welches gegebenenfalls durch Fluor, Cyano und/oder Ethinyl substituiert ist) substituiertes $C_3-C_6$-Cycloalkyl, für gegebenenfalls durch Chlor substituiertes Benzyl oder Phenylethyl, oder für gegebenenfalls durch Halogen, $C_1-C_4$-Alkyl und/oder $C_1-C_4$-Alkoxy substituiertes Phenyl steht,
$R^2$ für Chlor, Brom, Alkyl, Alkenyl oder Alkinyl, jeweils mit bis zu 5 C-Atomen oder für Phenyl, welches gegebenenfalls durch Halogen, $C_1-C_4$-Alkyl, $C_1-C_2$-Halogenalkyl,$C_1-C_4$-Alkoxy,$C_1-C_2$-Halogenalkoxy, $C_1-C_2$-(Halo)Alkylendioxy, $C_1-C_4$-Alkylthio und/oder $C_1-C_2$-Halogenalkylthio substituiert ist, steht,
X für Halogen steht,

bei welchem man $\alpha$-Hydroxy-phosphonsäureester der Formel II

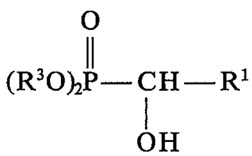

(II)

in welcher

$R^1$ die oben angegebene Bedeutung hat und
$R^3$ für Alkyl oder Phenyl steht oder die beiden Reste $R^3$ zusammen für geradkettiges oder verzweigtes Alkandiyl (Alkylen) stehen,

mit phosphorhaltigen Olefinierungsmitteln der Formel III

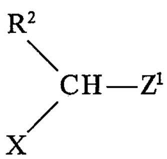

(III)

in welcher

$R^2$ und X die oben angegebenen Bedeutungen haben und
$Z^1$ für einen phosphorhaltigen Rest

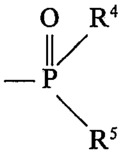 oder $-P(R^6)_3^{\oplus}$ $X^{\ominus}$

steht, worin

$R^4$ und $R^5$ gleich oder verschieden sind und einzeln für Alkyl, Phenyl, Alkoxy oder Phenoxy oder zusammen für Alkandioxy stehen,
$R^6$ für Alkyl oder Phenyl steht und
X für Halogen steht,

in Gegenwart von Basen, gegebenenfalls in Gegenwart von Katalysatoren, und gegebenenfalls unter Verwendung von Verdünnungsmitteln bei Temperaturen zwischen $-70$ und $+150°$ C umsetzt, dadurch gekennzeichnet, daß man in Gegenwart von Halogenüberträgern arbeitet.

2. Verfahren gemäß Anspruch 1, dadurch gekennzeichnet, daß als Halogenüberträger Tetrachlorkohlenstoff verwendet wird.

**Claims**

1. Process for the preparation of halogenoalkenes of the formula

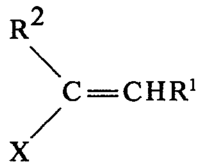

(I)

in which

$R^1$ represents optionally halogen-substituted $C_1-C_4$-alkyl, $C_2-C_6$-alkenyl, $C_3-C_6$-cycloalkyl which is optionally substituted by halogen, cyano, carbamoyl, $C_1-C_4$-alkyl, $C_1-C_4$-alkyl-carbonyl, $C_1-C_4$-alkoxycarbonyl and/or phenoxybenzyloxy-carbonyl (which is optionally substituted by fluorine, cyano and/or ethinyl), or phenylethyl or benzyl, optionally substituted by chlorine, or phenyl which is optionally substituted by halogen, $C_1-C_4$-alkyl and/or $C_1-C_4$-alkoxy,

$R^2$ represents chlorine, bromine, alkyl, alkenyl or alkinyl, in each case with up to 5 C atoms, or phenyl which is optionally substituted by halogen, $C_1-C_4$-alkyl, $C_1-C_2$-halogenoalkyl, $C_1-C_4$-alkoxy, $C_1-C_2$-halogenoalkoxy, $C_1-C_2$-(halogeno)alkylenedioxy, $C_1-C_4$-alkylthioand/or$C_1-C_2$-halogenoalkylthio,

X represents halogen,

in which $\alpha$-hydroxy-phosphonic acid esters of the formula II

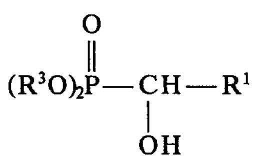

(II)

in which

$R^1$ has the meaning indicated above and

$R^3$ represents alkyl or phenyl or the two radicals $R^3$ together represent straight-chain or branched alkanediyl (alkylene),

are reacted with phosphorus-containing olefinating agents of the formula III

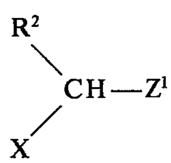

(III)

in which

$R^2$ and X have the meanings indicated above and

$Z^1$ represents a phosphorus-containing radical

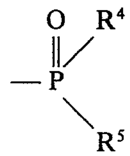 or $-P(R^6)_3^{\oplus}$ $X^{\ominus}$

wherein

$R^4$ and $R^5$ are identical or different and individually represent alkyl, phenyl, alkoxy or phenoxy or together represent alkanedioxy,
$R^6$ represents alkyl or phenyl and
X represents halogen,

in the presence of bases, optionally in the presence of catalysts and optionally using diluents, at temperatures of between $-70$ and $+150°C$, characterized in that the reaction is carried out in the presence of halogen carriers.

2. Process according to claim 1, characterized in that carbon tetrachloride is used as the halogen carrier.

**Revendications**

1. Procédé de fabrication d'halogéno-alcènes de formule I

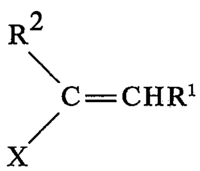

(I)

dans laquelle

$R^1$ représente un alcoyle en $C_1-C_4$ éventuellement halogéno-substitué, un alcényle en $C_2-C_6$, un cycloalcoyle en $C_3-C_6$ éventuellement substitué par de l'halogène, cyano, carbamoyle, alcoyle en $C_1-C_4$, alcoyle $(C_1-C_4)$-carbonyle, alcoxy$(C_1-C_4)$-carbonyle et/ou phénoxybenzyloxy-carbonyle (qui est éventuellement substitué par du fluor, cyano et/ou éthinyle, un benzyle ou phényléthyle éventuellement substitué par du chlore ou un phényle éventuellement substitué par de l'halogène, alcoyle en $C_1-C_4$ et/ou alcoxy en $C_1-C_4$,
$R^2$ représente du chlore, brome, alcoyle, alcényle ou alcinyle ayant chacun jusqu'à 5 atomes de carbone ou phényle qui est éventuellement substitué par de l'halogène, alcoyle en $C_1-C_4$, halogénoalcoyle en $C_1-C_2$, alcoxy en $C_1-C_4$, halogéno-alcoxy en $C_1-C_2$, (halo)alcoylènedioxy en $C_1-C_2$, alcoylthio en $C_1-C_4$ et/ou halogénoalcoylthio en $C_1-C_2$,
X représente de l'halogène,

dans lequel on fait réagir des esters d'acides $\alpha$-hydroxy-phosphoniques de formule II:

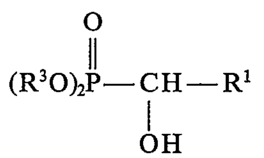

(II)

dans laquelle

$R^1$ a la signification indiquée plus haut et les
$R^3$ représentent un alcoyle ou phényle, ou les deux radicaux $R^3$ représentent ensemble un alcanediyle (alcoylène) à chaîne droite ou ramifiée,

avec des agents d'oléfination renfermant du phosphore de formule III:

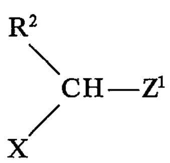

(III)

dans laquelle

$R^2$ et X ont les significations indiquées plus haut et
$Z^1$ représente un radical renfermant du phosphore:

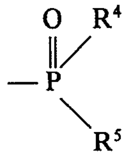 ou $-P(R^6)_3^{\oplus}$ $X^{\ominus}$

où

$R^4$ et $R^5$ sont identiques ou différents et représentent individuellement un alcoyle, phényle, alcoxy ou phénoxy, ou ensemble un alcanedioxy,

$R^6$ représente un alcoyle ou phényle et

X représente de l'halogène,

en présence de bases, éventuellement en présence de catalyseurs et éventuellement avec utilisation de diluants à des températures entre $-70$ et $+150°C$, caractérisé en ce qu'on opère en présence de transmetteurs d'halogène.

2. Procédé selon la revendication 1, caractérisé en ce qu'en tant que transmetteur d'halogène on utilise du tétrachlorure de carbone.